# EUROPÄISCHE PATENTSCHRIFT

(11) **EP 0 738 493 B1**
(45) Veröffentlichungstag und Bekanntmachung des Hinweises auf die Patenterteilung: **24.02.1999**
(21) Anmeldenummer: 96105767.6
(22) Anmeldetag: 12.04.1996
(51) Int. Cl.: A47L 15/46

(54) **Programmgesteuerte Geschirrspülmaschine, insbesondere Spülautomat für den Labor- und Krankenhausbereich**
Programme actuated dishwashing machine, in particular rinse machine for hospital and laboratory premises
Lave-vaiselle programmable, en particulier appareil de rinçage pour le domaine du laboratoire et de l'hôpital

(30) Priorität: 22.04.1995 DE 19514873
(43) Veröffentlichungstag der Anmeldung: 23.10.1996
(73) Patentinhaber: Miele & Cie. GmbH & Co., D-33332 Gütersloh (DE)
(72) Erfinder: Hein, Wolfgang, 33613 Bielefeld (DE); Hettenhausen, Ulrich, 33739 Bielefeld (DE); Kethers, Frank, 32791 Lage/Lippe (DE); Obermeier, Peter, 33699 Bielefeld (DE)

(56) Entgegenhaltungen:
- EP-A- 0 619 395
- DE-C- 3 516 006
- FR-A- 2 145 653
- US-A- 4 655 170

## Beschreibung

Der Gegenstand der Erfindung betrifft eine programmgesteuerte Geschirrspülmaschine, mit einem oder mehreren Einsatzwagen oder Spülgutkörben insbesondere einen Spülautomaten für den Labor- und Krankenhausbereich, zum programmgesteuerten Reinigen, Desinfizieren und Trocknen von Spülgut, wie Atemschläuche, Katheter, OP-Instrumente, Pipetten oder dergl., welches für die Behandlung im türverschlossenen Spülraum des Automaten in speziellen Einsatzwagen oder separaten Spülgutkörben einzusortieren ist, wobei jeder Wagen oder Korb mit einem Magnetschlüssel versehen ist, dessen Magnetisierung das Spülprogramm für das im Korb geladene Spülgut bestimmt.

Die DE-OS 27 01 879 beschreibt eine Geschirrspülmaschine, bei der am Geschirrkorb hinten ein Schlüssel zur Einstellung des Spülprogramms angeordnet ist. Dieser Schlüssel besteht aus einem Magnetstab, dessen Magnetisierung das jeweilige Spülprogramm bestimmt. Die in dieser Spülmaschine eingesetzten Spülgutkörbe sind jeweils zur Aufnahme einer bestimmten Spülgutart ausgebildet. Durch die spezielle Korbausbildung für bestimmte Geschirrarten in Verbindung mit dem Magnetschlüssel ist sichergestellt, daß das richtige Programm auch ausgewählt wird. Dafür ist dem Magnetstab eine an der hinteren Spülbehälterwand befindliche Schloßmatrix zugeordnet, welche bei eingeschobenem Korb oder Spülgutwagen die Magnetisierung des Magnetstiftes abfragt. Bei diesem Technikstand ist es als Nachteil anzusehen , daß bei einer Änderung des Spülprogramms der Magnetstab komplett gegen einen anders codierten Stab ausgewechselt werden muß. Hierdurch wird eine Vielzahl von Einzelmagnetstäben notwendig.

Aus der DE-PS 35 16 006 ist es ferner bei einer Wasch- und Desinfektionsanlage bekannt, zur individuellen Programmeinstellung Einzelmagneten in einer vorgegebenen Kombination am Wagen anzuordnen, wobei je nach Kombination Dauermagnete hinzugefügt oder entfernt werden. Die einzeln montier- und demontierbaren Dauermagnete sind dabei entweder seitlich am Wagengestell oder stirnseitig desselben und der Spülraumrückwand zugewandt angeordnet. Die Magnete schalten mehrere im Bereich der hinteren oder seitlichen Spülbehälterwand der Anlage aussenseitig angeordnete Reedkontakte. Nachteilig hierbei ist, daß die Anordnung der Dauermagnete an der hinteren oder seitlichen Wagenseite eine entsprechende Anordung von Magnetschaltern (Reedkontakte) an der hinteren oder seitlichen Spülbehälteraußenseite erforderlich macht. Da Spülautomaten im Labor- oder OP-Bereich nun im Regelfall hinten von der angrenzenden Raumwand und seitlich von weiteren dicht anstehenden Geräten oder Schränken begrenzt sind, ist eine solche Anordnung der Elemente zur Programmwahl keinesfalls als servicefreundlich anzusehen. Für einen Austausch oder eine kontrollierbare Nachstellung der Schaltelemente müßte der schwere Spülautomat mühevoll aus seiner Installationsnische hervorgezogen werden, was sehr aufwendig und aus zeit- und kostengründen kaum vertretbar ist. Die Anordnung von Einzelmagneten am Korb oder Wagen verlangt ferner entsprechende Einzelhalterungen, die eine rationelle Korb-oder Wagenfertigung bei verschiedenartig auszubildenden Körben oder Einsatzwagen erschweren. Auch können einzeln montierbare Dauermagneten sich vom Wagen lösen oder verloren gehen. Darüber hinaus besitzen Reedkontakte keine exakt definierten Schaltpunkte und reagieren empfindlich auf Streufelder, so daß ggf. Programmfehlsteuerungen ausgelöst werden können. Es ist insbesondere beim maschinellen Spülen und Reinigen und ggf. Desinfizieren von im OP-Bereich oder im Labor eingesetztem Spülgut sicherzustellen, daß keine falschen Spülprogramme ausgelöst werden. Falsch aufgerufene Programme sind mit Fehlbedienungen gleichzusetzen, die u. U. empfindliches oder wertvolles Spülgut zerstören können. Bei der bekannten Steuerung ist auch keinerlei Kontrolle über die Richtigkeit der von den Magneten bzw. Reedkontakten erzeugten Signale gegeben.

Ausgehend von einer programmgesteuerten Geschirrspülmaschine der eingangs genannten Art liegt der Erfindung die Aufgabe zugrunde, die Nachteile des bekannten Technikstandes zu vermeiden und ohne einzeln montier- sowie demontierbare Dauermagneten eine individuelle Programmwahl für den eingeschobenen Spülgutwagen oder -korb einfach und schnell zu treffen. Auch soll sichergestellt werden, daß kein falsches Programm zum Ablauf gebracht wird, wenn an der Programmauswahl beteiligte Bauelemente ausfallen oder falsch schalten.

Die Erfindung löst diese Aufgabe durch die Merkmale des Anspruchs 1. Vorteilhafte Weiterbildungen sind den Unteransprüchen zu entnehmen.

Die mit der Erfindung erzielten Vorteile liegen insbesondere darin, daß die Einsatzwagen oder Spülgutkörbe einfach und schnell auf bestimmte Reinigungsprogramme oder dergl. einstellbar sind, wobei pro Wagen oder Korb die gleichen Magnetschlüssel mehrfach genutzt werden können. Ferner ist durch den Einsatz von Magnetsensoren, welche gezielt Polaritätsausrichtungen der Magnetschlüssel erkennen können, sichergestellt, daß kein falsches Programm durch einen Korb oder Wagen angewählt werden kann, so daß eine große Sicherheit für das aufzubereitende Spülgut gegeben ist.

Die nachstehende Beschreibung dient der Erläuterung des Gegenstands gemäß der Erfindung, von dem ein Ausführungsbeispiel in den Zeichnungen dargestellt ist. Es zeigen:
- Figur 1: einen mit einem Einsatzwagen für aufzubereitendes Spülgut beladenen Spülautomaten mit geöffnetem Spülraum, in der Seitenansicht,
- Figur 2: einen Magnetschlüssel für den Einsatzwagen in Form einer Magnetleiste in der Draufsicht,
- Figur 3: den Magnetschlüssel in Einbaulage am Einsatzwagen,
- Figur 4: von den Magnetschlüssels der Einsatzwagen aktivierbare Magnetsensoren am Spülautomaten,
- Figur 5: einen Permanentmagneten des Magnetschlüssels am Einsatzwagen in Wirkstellung zu einem Magnetsensor am Spülautomaten, ausschnittsweise in schematischer Darstellung,
- Figur 6: eine Codetabelle über abprüfbare Codemuster zur Programmauswahl, die von den Magnetsensoren erzeugt werden,
- Figur 7: eine weitere Codetabelle über abprüfbare Codemuster zur Programmauswahl.

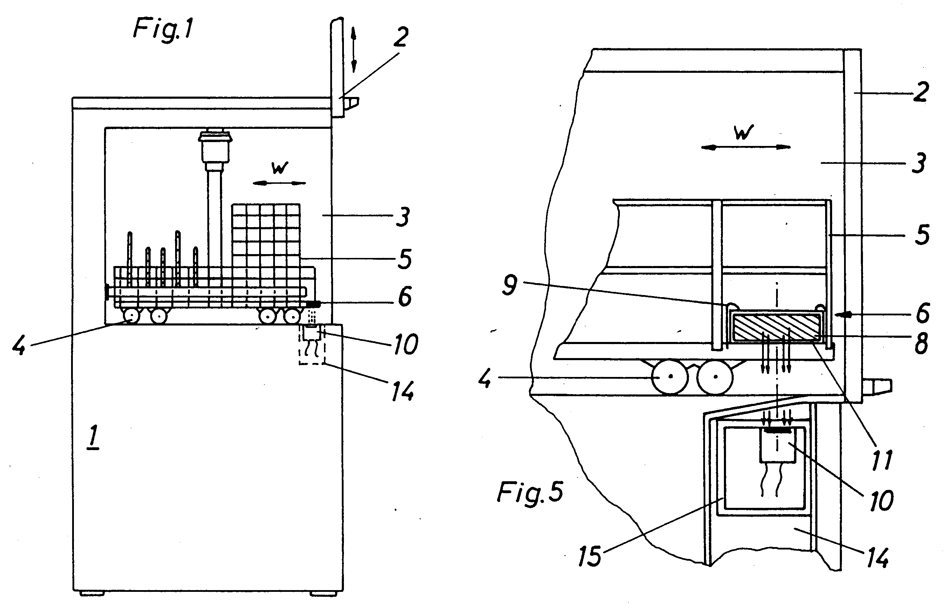

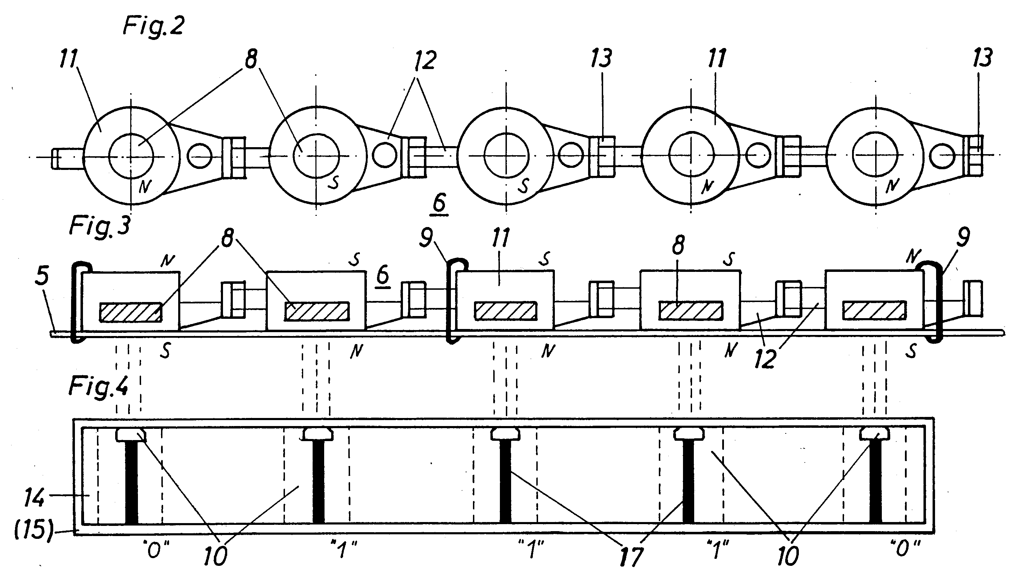

Ein in Fig. 1 mit (1) bezeichneter Spülautomat, insbesondere für den Labor-und Krankenhausbereich, weist einen durch eine Gerätetür (2) - im Ausführungsbeispiel eine vertikal verschiebbare Hubtür - verschließbaren Spülraum (3), zum programmgesteuerten Reinigen, Desinfizieren und Trocknen von Spülgut, wie Atemschläuche, Katheter, OP-Instrumente, Pipetten oder dergl. auf. Dieses Spülgut (nicht dargestellt) wird in einem in bekannter Weise mit Laufrädern (4) versehenen Spülguteinsatz oder Einsatzwagen (5) für die Behandlung im Gerät einsortiert. Für die Behandlung oder Aufbereitung des Spülgutes im Spülautomaten (1) stehen mehrere separat aufrufbare Reinigungs- und Desinfektionsprogramme zur Verfügung, welche spülgut- bzw. einsatzwagenabhängig auswählbar sind. Entsprechend sind auch mehrere Einsatzwagentypen notwendig. Hierfür ist jeder eine bestimmte Art von Spülgut aufnehmender Einsatzwagen (5) oder Spülgutkorb mit einem Magnetschlüssel (6), Fig. 2, versehen, dessen Magnetisierung das Spülprogramm für das im Korb geladene Spülgut bestimmt. Die Magnetisierung der Schlüssel ist bei abgenommenem Magnetschlüssel (6) veränderbar.

Der Magnetschlüssel (6) ist am gerätetürseitigen Wagen- (7) oder Korbgestell, sh. Fig. 1, 3 und 5, befestigt und als einstückige flache Magnetleiste aus mehreren separaten Permanentmagneten (8) in einer wählbaren oder änderbaren, für einen bestimmten Einsatzwagentyp aber vorgegebenen Einzelpolaritätsausrichtung (Nord-Süd/Süd-Nord) ausgebildet. Für jeden Korb oder Wagen ist vorzugsweise eine gleiche Anzahl von Permanentmagneten (8) vorgesehen, die jedoch entsprechend dem jeweils auszuwählenden Spülprogramm in ihrer Einzelpolaritätsausrichtung unterschiedlich gepolt sind, wie dies bspw. in den Fig. 2 und 3 gezeigt ist. Jeder Magnetschlüssel (6) ist zur Änderung der Einzelpolaritätsausrichtung der Magnete türseitig vorn am Einsatzwagen (5) oder Spülgutkorb abnehmbar befestigt. Für die lösbare Befestigung des Magnetschlüssels (6) am Wagen können Halteklammern (9) oder dergl., die vorzugsweise als separate Klammern ausgebildet sind, verwendet werden. Dies erspart Änderungen an bestehenden noch nicht mit Magnetschlüsseln (6) ausgerüsteten Serien-Einsatzwagen.

Die Permanentmagnete (8) einer Magnetleiste sind nach Art einer Kette innerhalb des Magnetschlüssels (6) aneinandergereiht und innerhalb der Kettenverbindung zur leichten Änderung ihrer Einzelpolaritätsausrichtung (Nord-Süd/Süd-Nord) in Bezug auf die diese Polarität erkennenden Magnetsensoren (10) jeweils um sich selbst als separate Kettenglieder drehbar angeordnet. Die Einsatzwagen (5) oder Spülgutkörbe lassen sich dadurch einfach und schnell auf bestimmte Reinigungs-oder Spülprogramme codieren, wobei pro Wagen oder Korb die gleichen Magnetschlüssel (6) mehrfach genutzt werden können. Vorzugsweise sind die separaten Kettenglieder nicht trennbar in den Magnetschlüsseln (6) angeordnet, so daß jeder Schlüssel als Ganzes betrachtet werden kann, bei dem die einzelnen Permanentmagnete (8) nicht verloren gehen oder sich vom Wagen lösen können. Natürlich ist auch eine Ausbildung von Magnetschlüssels (6) mit wiederlösbaren Kettengliedern möglich. Zweckmäßig wären hierfür Sicherheitsverschlüsse zwischen den einzelnen Kettengliedern.

Jeder Permanentmagnet (8) einer Magnetleiste wird von einer Halterung (11) bspw. aus Kunststoff mit beidseitig angeformten Kupplungselementen (12) aufgenommen. Die Kupplungselemente (12) greifen um 180° oder 360° drehbar ineinander. Eine besonders einfache Verbindungart ergibt sich, wenn die Einsätze über Rastverbindungen (13) miteinander verkettet sind. Die von den Halterungen (11) aufgenommenen Permanentmagnete (8) sind gemäß Fig. 3 bis 5 scheibenförmig ausgebildet, so daß sie flach am nichtmagnetischen in der Regel aus Edelstahl gefertigtem Einsatzwagengestell oder Spülgutkorb anliegen können. Hierdurch ist auch ein gleichbleibender Abstand zu den benachbarten Magnetsensoren (10) gegeben.

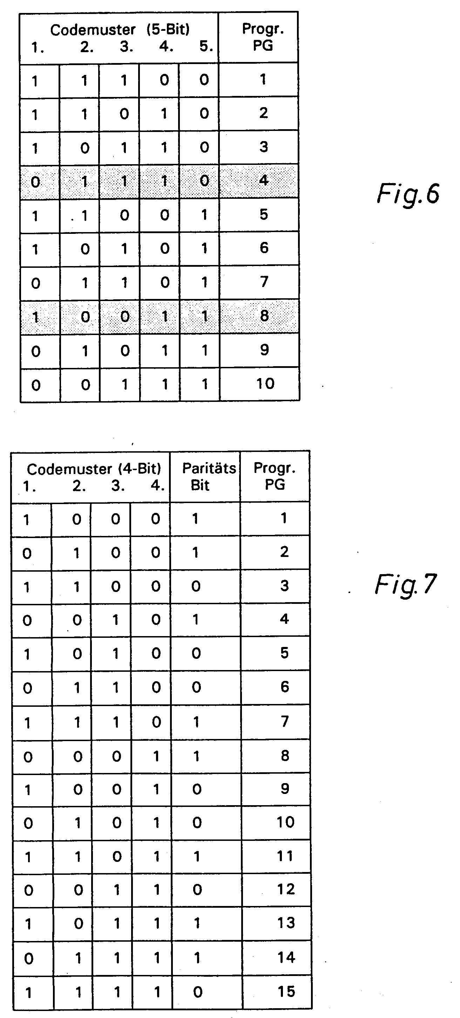

Die Anordnung der Magnetleisten oder Magnetschlüssel (6) am Einsatzwagengestell (7) ist so gewählt, daß die Magnetfeldausrichtung der Magnetpole für die aktivierbaren Magnetsensoren (10) quer zur Verschieberichtung (Pfeil W, Fig. 1 oder 5) des Einsatzwagens (5) oder Spülgutkorbes verläuft. Dafür sind die von den Magneten aktivierbaren Magnetsensoren (10) jeweils außerhalb des Spülraumes (3) in der Gerätetür (2) oder unterhalb der Spülraumöffnung (Fig. 4 und 5) im Bereich der Gerätetür (2) in einem dafür vorgesehenen Raum (14) vorgesehen, der ggf. hinter der Frontverkleidung der Spülmaschine erreichbar ist. Die Magnetsensoren (10) erzeugen den Magnetpolaritäten des Magnetschlüssels (6) entsprechend abprüfbare Codemuster zur Spülprogrammauswahl. Wenn die Programmvielfalt ggf. einmal erweitert werden soll, so ist es auch möglich, die Anzahl der Magnetsensoren gleich schon größer als die Zahl der Permanentmagnete der jeweils eingesetzten Magnetschlüssels zu wählen. Im Ausführungsbeispiel entspricht die Anzahl der Magnetsensoren (10) der Anzahl der Permanentmagnete (8) eines Magnetschlüssels (6).

Eine besonders servicefreundliche Unterbringung der Magnetsensoren (10) ergibt sich, wenn diese vorzugsweise in einer frontseitigen Hohlleiste (15) (Fig. 2) im Raum (14) unterhalb des Spülraumbodens (16) montiert sind. Hierdurch ist es bei abgenommener Frontverkleidung des Spülautomaten einfach möglich, Lagekorrekturen des Abtastsystems kontrollierbar durchzuführen, da eine Einstellung der Magnetsensoren (10) immer mit Blick auf die jeweilige Lage bzw. Ausrichtung der Magnetleiste am eingeschobenen Einsatzwagen (5) und umgekehrt durchgeführt werden kann.

Bei der Verwendung einer frontseitig klappbaren Gerätetür (nicht gezeigt) können die Magnetsensoren (10) innerhalb der Gerätetür zwischen dem Türinnenblech und dem Türaußenblech positioniert werden. Auch eine solche Montage ist sehr servicefreundlich. Bei geschlossener Gerätetür kommen die Magnetsensoren (10) dann in den Wirkbereich der Permanentmagneten (8) des Magnetschlüssels (6), so daß das Codemuster des Wagens bei Programmbeginn abgefragt und vom System abgeprüft werden kann.

Vorteilhaft sind als Magnetsensoren (10) von den Magnetschlüsseln (6) angesteuerte Hallsensoren verwendet, welche die Einzelpolaritätsausrichtung (Nord-Süd/Süd-Nord) der Permanentmagnete (8) direkt erkennen und den Magnetpolaritäten entsprechend abprüfbare Codemuster zur Spülprogrammauswahl erzeugen. Diese Hallsensoren, die gegenüber den bekannten Reedkontakten einen exakt definierten Schaltpunkt besitzen und auch in höheren Temperaturbereichen sicherer arbeiten, können so ausgelegt sein, daß sie entweder den Nordpol "N" eines Permanentmagneten (8) des Magnetschlüssels (6) als log "1" (logisch "Eins") erkennen oder aber umgekehrt als log "0" (logisch "Null"). Für die hier beschriebene Ausführung der Magnetsensoren (10) wird log "1" als Signal erzeugt. In diesem Fall ist der Nordpol "N" des Permanentmagneten (8) zum Magnetsensor (10) ausgerichtet und der Südpol "S" des Permanentmagneten (8) zeigt im Einsatzwagen (5) nach oben, vergl. Fig. 3 und 4 bzw. 5. Gegenüber den bekannten Reedkontakten als Magnetschalter, welche immer schalten, wenn ein magnetisches Kraftfeld oder gar ein unerwünschtes Streufeld ansteht, egal von welcher Flußrichtung es ist, bzw. von welchem Magnetpol (Nord- oder Südpol) es kommt, können Hallsensoren als Magnetsensoren so eingestellt sein, daß sie eine bestimmte Polarität erkennen und hieraus direkt durch log "1" oder log "0" reagieren. In Kombination mit den sie beeinflussenden Magnetschlüsseln können somit leicht und einfach die abprüfbaren Codemuster erzeugt werden.

Als Magnetsensoren (10) mit der gleichen Wirkung könnten jedoch auch nicht dargestellte Mikroschalter eingesetzt werden, deren Schaltwippen Einzelmagnete tragen, oder deren Schaltwippen direkt durch Magnetkräfte beeinflußbar sind. Bei der Verwendung von Einzelmagneten werden diese bspw. alle so polarisiert, daß ihre Nordpole "N" in Richtung des am Einsatzwagen (5) befestigten Magnetschlüssels (6) zeigen. Hierbei ist darauf zu achten, daß bspw. die Rückstellkraft der Wippe größer als die Gewichtskraft des Magneten ist. In diesem Fall bleibt der Mikroschalter bei nicht angesteuertem Magnetsensor (10) offen (log "0" oder logisch "Null"). Ein Schaltvorgang wird durch die sich abstoßenden Magnetkräfte der sich gegenseitig beinflussenden Magnetfelder des Permanentmagneten (8) am Einsatzwagen (5) und des Magneten am Mikroschalter ausgelöst. Auch hier erkennt der Magnetsensor (10) die Einzelpolaritätsausrichtung des ihm zugeordneten Permanentmagneten (8). Trifft ein Nordpol "N" des Magneten der Magnetleiste auf einen Nordpol "N" am Mikroschalter, so wird der Mikroschalter betätigt und erzeugt ein Signal log "1", welches in Kombination mit den Signalen der anderen Mikroschalter das abprüfbare Codemuster zur Spülprogrammauswahl erzeugt. Entsprechend umgekehrt kann aber auch die Rückstellkraft der Wippe kleiner als die Gewichtskraft des Magneten sein. In diesem Fall ist der Mikroschalter bei nicht angesteuertem Magnetsensor (10) geschlossen (log "1"). Ein Schaltvorgang wird dann durch die sich anziehenden Magnetkräfte der Magnetfelder ausgelöst.

Durch die über die Magnetschlüssel (6) aktivierten Magnetsensoren (10), wie Hallsensoren oder Mikroschalter mit durch Magnetkräfte betätigbaren Schaltwippen sind Codemuster (sh. auch Fig. 4 und 6, 7) entsprechend der Anzahl wählbarer Spülprogramme oder dergl. erzeugbar. Dies erzeugten Codemuster sind abprüfbar, wofür Vergleichscodemuster in dem nicht gezeigten Programmspeicher des Spülautomaten (1) abrufbar abgespeichert sind. Das Abprüfen erfogt jeweils vor dem Programmstart. Damit immer das richtige Programm von einem Magnetschlüssel (6) aufgerufen wird, werden die von den Magnetschlüsseln (6) in einem Digitalcode erzeugten Codemuster vor Programmfreigabe mit den abgespeicherten Mustern verglichen und nur bei Übereinstimmung freigegeben.

Die Prüfung der Codemuster auf Richtigkeit genügt bestimmten Regeln. So ist bspw. die Prüfvorschrift derart, daß Codemuster nur immer dann gültig sein können, wenn jeweils eine gleiche Anzahl von log "1" oder log "0" erzeugt wird. Ein Abweichen von dieser Vorschrift wird als Fehlermeldung gewertet. Dabei wird davon ausgegangen, daß die Magnetleisten alle die gleiche Anzahl von Magneten, z. B. fünf Permanentmagnete (8) (entspr. fünf Magnetsensoren (10)), aufweist.

Entsprechend erzeugbare gültige Codemuster im 5-Bit-Codewort zeigt hierfür die Tabelle gemäß Fig. 6. Gemäß dieser Tabelle sind 10 verschiedene Spülgut-Aufbereitungsprogramme durch die Einsatzwagen (5) bzw. deren Magnetschlüssel (6) auswählbar. Die Fehlersicherheit wird vorteilhaft also dadurch erreicht, daß nur immer Codemuster gültig sind, die eine gleiche Anzahl von log "1" besitzen; in diesem Fall nur Codemuster, welche 3 mal den Wert log "1" besitzen. Wenn nun innerhalb eines Codewortes log "1" zu log "0" umkippt, dann kann es sich nur um einen Fehlerzustand handeln. Die Summe der erzeugten log "1" ist ungleich drei. Ein solches Codemuster wird vom System erkannt und nicht zugelassen. Auch der umgekehrte Fall, wenn bei den Magnetsensoren (10) log "0" zu log "1" wird, löst ebenfalls einen Fehler aus, welcher den Programmstart verhindert.

Die abprüfbaren Codemuster können gemäß Fig. 7 auch der Vorschrift genügen, daß die z. B. im 4-Bit-Codewort erzeugten Codemuster durch ein Paritätsbit ergänzt werden. Dieses Paritätsbit wird zu log "1", wenn die Summe von log "1" ungerade und zu log "0", wenn die Summe von log "1" gerade ist. Die Fig. 7 zeigt die Möglichkeit der Auswahl von insgesamt 15 Programmen bzw. Codemustern, wobei ein 4-Bit-Codewort um ein Paritätsbit (log "1" oder log "0") ergänzt ist.

In der Fig. 3 ist eine Einzelpolaritätsausrichtung "S" - "N" - "N" - "N" - "S" der Permanentmagnete (8) eines Magnetschlüssels (6) gezeigt, welches ein 5-Bit-Codemuster nach der Bitkombination log "0" - log "1" - log "1" - log "1" - log "0" erzeugt und bspw. das 4. Programm (Spülgut-Aufbereitungsprogramm PG 4) auswählt, sh. auch Tabelle in Fig. 6. Dieses Codemuster wird von fünf Magnetsensoren (10), Fig. 4, erzeugt.

Die Einzelpolaritätsausrichtung "N" - "S" - "S" - "N" - "N" nach Fig. 2, wenn die in der Draufsicht gezeigte Polarität die Magnetsensoren beeinflussen würde, hingegen wird ein abprüfbares Codemuster aus der Bitkombination log "1" - log "0" - log "0" - log "1" - log "1" erzeugen, welche z. B. das 8. Programm aus der Tabelle gemäß Fig. 6 (PG 8) auswählt.

Damit sichergestellt ist, daß die Magnetsensoren (10) den Magnetfeldern der Magnetleisten an den Einsatzwagen (5) oder Spülgutkörben auch optimal ausgesetzt sind, bzw. auf diese auch sicher ansprechen, ist zur Fokussierung der Magnetfelder jedem Sensor gemäß Fig. 4 noch ein Fokussierblech (17) zugeordnet. Hierdurch kann auch einer eventuelle Fremdbeeinflussung der Magnetfelder z. B. durch magnetisch leitendes Spülgut wirksam vorgebeugt werden.

Es versteht sich, daß die die erfindungsgemäßen Magnetschlüssel in Funktion mit den die jeweilige Polarität der Einzelmagneten erkennenden Magnetsensoren ebenso für Geschirrspülmaschinen im Haushaltbereich vorteilhaft einsetzbar sind. Anwendungsfälle hierfür ergeben sich beispielsweise, wenn Spezialkörbe oder Spülguteinsätze, z. B. ein Unterkorb für besonderes Topfgeschirr oder ein Oberkorb für empfindliche Gläser, welche besonders schonend gespült werden müssen, individuelle korb- oder spülgutbezogene Programme aufrufen sollen.

## Patentansprüche

1. Programmgesteuerte Geschirrspülmaschine, mit einem oder mehreren Einsatzwagen oder Spülgutkörben insbesondere Spülautomat für den Labor- und Krankenhausbereich, zum programmgesteuerten Reinigen, Desinfizieren und Trocknen von Spülgut, wie Atemschläuche, Katheter, OP-Instrumente, Pipetten oder dergl., welches für die Behandlung im türverschlossenen Spülraum des Automaten in speziellen Einsatzwagen oder separaten Spülgutkörben einzusortieren ist, wobei jeder Wagen oder Korb mit einem Magnetschlüssel versehen ist, dessen Magnetisierung das Spülprogramm für das im Korb geladene Spülgut bestimmt,
dadurch gekennzeichnet,
daß der Magnetschlüssel (6) am Einsatzwagen oder Spülgutkorb (5) als Magnetleiste aus mehreren Permanentmagneten (8) in einer änderbaren Einzelpolaritätsausrichtung (Nord-Süd/Süd-Nord) ausgebildet ist.

2. Programmgesteuerte Geschirrspülmaschine, insbesondere Spülautomat für den Labor- und Krankenhausbereich, nach Anspruch 1,
dadurch gekennzeichnet,
daß der Magnetschlüssel (6) am gerätetürseitigen Wagen- oder Korbgestell (7) befestigt und zur Spülprogrammänderung vom Einsatzwagen (5) oder Spülgutkorb abnehmbar ausgebildet ist, und daß in der Gerätetür (2) oder unterhalb der Spülraumöffnung im Bereich der Gerätetür (2) durch die Magnetschlüssel (6) aktivierbare und die jeweilige Einzelpolarität der Permanentmagnete (8) eines Schlüssels erkennende Magnetsensoren (10) vorgesehen sind.

3. Programmgesteuerte Geschirrspülmaschine, insbesondere Spülautomat für den Labor- und Krankenhausbereich, nach Anspruch 1 und 2,
dadurch gekennzeichnet,
daß die Magnetsensoren (10) den Magnetpolaritäten der Permanentmagnete (8) des Magnetschlüssels (6) entsprechende abprüfbare Codemuster zur Spülprogrammauswahl erzeugen.

4. Programmgesteuerte Geschirrspülmaschine, insbesondere Spülautomat für den Labor- und Krankenhausbereich, nach einem oder mehreren der Ansprüche 1 bis 3,
dadurch gekennzeichnet,
daß Magnetsensoren (10) in gleicher Anzahl wie Permanentmagnete (8) eines Magnetschlüssels (6) vorhanden sind.

5. Programmgesteuerte Geschirrspülmaschine, insbesondere Spülautomat für den Labor- und Krankenhausbereich, nach einem oder mehreren der Ansprüche 1 bis 4,
dadurch gekennzeichnet,
daß für jeden Korb oder Wagen eine gleiche Anzahl von Permanentmagneten (8) vorgesehen ist, die jedoch entsprechend dem jeweils auszuwählenden Spülprogramm in ihrer Einzelpolaritätsausrichtung unterschiedlich gepolt sind.

6. Programmgesteuerte Geschirrspülmaschine, insbesondere Spülautomat für den Labor- und Krankenhausbereich, nach einem oder mehreren der Ansprüche 1 bis 5,
dadurch gekennzeichnet,
daß jeder Magnetschlüssel (6) abnehmbar am Einsatzwagen (5) oder Spülgutkorb befestigt ist.

7. Programmgesteuerte Geschirrspülmaschine, insbesondere Spülautomat für den Labor- und Krankenhausbereich, nach einem oder mehreren der Ansprüche 1 bis 6,
dadurch gekennzeichnet,
daß die Permanentmagnete (8) eines Magnetstreifens nach Art einer Kette innerhalb des Magnetschlüssels (6) aneinandergereiht und innerhalb der Kettenverbindung zur Änderung der Polaritätsausrichtung jeweils um sich selbst drehbar angeordnet sind.

8. Programmgesteuerte Geschirrspülmaschine, insbesondere Spülautomat für den Labor- und Krankenhausbereich, nach einem oder mehreren der Ansprüche 1 bis 7,
dadurch gekennzeichnet,
daß die Permanentmagnete (8) als separate Kettenglieder vorzugsweise nur bei vom Einsatzwagen (5) oder Spülgutkorb demontierter Magnetleiste in ihrer Einzelpolaritätsausrichtung veränderbar sind.

9. Programmgesteuerte Geschirrspülmaschine, insbesondere Spülautomat für den Labor- und Krankenhausbereich, nach einem oder mehreren der Ansprüche 1 bis 8,
dadurch gekennzeichnet,
daß die Permanentmagnete (8) als vorzugsweise unlösbare separate Kettenglieder in der Magnetleiste angeordnet sind.

10. Programmgesteuerte Geschirrspülmaschine, insbesondere Spülautomat für den Labor- und Krankenhausbereich, nach einem oder mehreren der Ansprüche 1 bis 9,
dadurch gekennzeichnet,
daß jeder Permanentmagnet (8) eines Magnetschlüssels (6) in einem nichtmagnetischem Einsatz mit beidseitig ausgebildeten Kupplungselementen (12) angeordnet ist, und daß die Kupplungselemente (12) um 180° drehbar ineinandergreifen.

11. Programmgesteuerte Geschirrspülmaschine, insbesondere Spülautomat für den Labor- und Krankenhausbereich, nach einem oder mehreren der Ansprüche 1 bis 10,
dadurch gekennzeichnet,
daß die Einsätze über Rastverbindungen (13) miteinander verkettet sind.

12. Programmgesteuerte Geschirrspülmaschine, insbesondere Spülautomat für den Labor- und Krankenhausbereich, nach einem oder mehreren der Ansprüche 1 bis 11,
dadurch gekennzeichnet,
daß die Permanentmagnete (8) scheibenförmig ausgebildet sind und die Anordnung der Magnetleiste im Korb oder Einsatzwagen (5) so gewählt ist, daß die Magnetfeldausrichtung der Magnetpole für die aktivierbaren Magnetsensoren (10) quer zur Verschieberichtung des Einsatzwagens (5) oder Spülgutkorbes verläuft.

13. Programmgesteuerte Geschirrspülmaschine, insbesondere Spülautomat für den Labor- und Krankenhausbereich, nach einem oder mehreren der Ansprüche 1 bis 12,
dadurch gekennzeichnet,
daß die Magnetsensoren (10) vorzugsweise in einer Hohlleiste (15) unterhalb des Spülraumbodens (16) oder innerhalb der Gerätetür (2) zwischen dem Türinnenblech und dem Türaußenblech positioniert sind.

14. Programmgesteuerte Geschirrspülmaschine, insbesondere Spülautomat für den Labor- und Krankenhausbereich, nach einem oder mehreren der Ansprüche 1 bis 13,
dadurch gekennzeichnet,
daß als Magnetsensoren (10) von den Magnetschlüsseln (6) angesteuerte Hallsensoren verwendet sind.

15. Programmgesteuerte Geschirrspülmaschine, insbesondere Spülautomat für den Labor- und Krankenhausbereich, nach einem oder mehreren der Ansprüche 1 bis 13,
dadurch gekennzeichnet,
daß als Magnetsensoren (10) Mikroschalter verwendet sind, deren Schaltwippen Einzelmagnete tragen oder deren Schaltwippen direkt durch Magnetkräfte beeinflußbar sind.

16. Programmgesteuerte Geschirrspülmaschine, insbesondere Spülautomat für den Labor- und Krankenhausbereich, nach einem oder mehreren der Ansprüche 1 bis 15,
dadurch gekennzeichnet,
daß den Magnetsensoren (10) Fokussierbleche (17) zur Magnetfelderhöhung im Sensorbereich zugeordnet sind.

17. Programmgesteuerte Geschirrspülmaschine, insbesondere Spülautomat für den Labor- und Krankenhausbereich, nach einem oder mehreren der Ansprüche 1 bis 16,
dadurch gekennzeichnet,
daß Magnetschlüssel (6) mit gleicher Anzahl von Permanentmagneten (8) als abprüfbare Codemuster jeweils nur Codes mit einer gleichen Anzahl von log "1" oder log "0" erzeugen, und daß ein Abweichen von dieser Vorschrift als Fehlermeldung gewertet wird.

18. Programmgesteuerte Geschirrspülmaschine, insbesondere Spülautomat für den Labor- und Krankenhausbereich, nach einem oder mehreren der Ansprüche 1 bis 17,
dadurch gekennzeichnet,
daß die abprüfbaren Codemuster durch ein Paritätsbit zu log "1" oder zu log "0" in gerader oder ungerader Parität ergänzt werden.

## Claims

1. Program-controlled equipment washing machine, having one or more insertable carriages or baskets for items to be washed, more especially an automatic washing machine for the laboratory and hospital sector, for the program-controlled cleaning, disinfecting and drying of items to be washed, such as breathing tubes, catheters, OP instruments, pipettes or the like, which items are to be sorted into specific insertable carriages or separate baskets for items to be washed for treatment in the washing chamber of the automatic machine, which chamber has a sealed door, each carriage or basket being provided with a magnetic key, the magnetisation of which determines the washing program for the item to be washed, which is loaded in the basket, characterised in that the magnetic key (6) on the insertable carriage or basket for items to be washed (5) is configured as a magnetic bar formed from a plurality of permanent magnets (8) in a variable orientation of individual polarity (north-south/south-north).

2. Program-controlled equipment washing machine, more especially an automatic washing machine for the laboratory and hospital sector according to claim 1, characterised in that the magnetic key (6) is mounted on the carriage or basket frame (7) adjacent the appliance door and is adapted to be removable from the insertable carriage (5) or basket for items to be washed in order to change the washing program, and in that magnetic sensors (10) are provided in the appliance door (2) or beneath the washing chamber opening in the region of the appliance door (2), said sensors being actuatable by the magnetic keys (6) and detecting the individual polarity of each of the permanent magnets (8) of a key.

3. Program-controlled equipment washing machine, more especially an automatic washing machine for the laboratory and hospital sector, according to claims 1 and 2, characterised in that the magnetic sensors (10) produce detectable code patterns, which correspond to the magnetic polarities of the permanent magnets (8) of the magnetic key (6), in order to select the washing program.

4. Program-controlled equipment washing machine, more especially an automatic washing machine for the laboratory and hospital sector, according to one or more of claims 1 to 3, characterised in that magnetic sensors (10) are present in the same number as permanent magnets (8) of a magnetic key (6).

5. Program-controlled equipment washing machine, more especially an automatic washing machine for the laboratory and hospital sector, according to one or more of claims 1 to 4, characterised in that an identical number of permanent magnets (8) is provided for each basket or carriage, but said magnets are differently poled in respect of the orientation of their individual polarity according to the actual washing program to be selected.

6. Program-controlled equipment washing machine, more especially an automatic washing machine for the laboratory and hospital sector, according to one or more of claims 1 to 5, characterised in that each magnetic key (6) is removably mounted on the insertable carriage (5) or basket for items to be washed.

7. Program-controlled equipment washing machine, more especially an automatic washing machine for the laboratory and hospital sector, according to one or more of claims 1 to 6, characterised in that the permanent magnets (8) of a magnetic strip are arranged in adjacent rows in the form of a chain internally of the magnetic key (6) and are each disposed internally of the chain connection so as to be rotatable about themselves in order to change the orientation of polarity.

8. Program-controlled equipment washing machine, more especially an automatic washing machine for the laboratory and hospital sector, according to one or more of claims 1 to 7, characterised in that the permanent magnets (8), as separate chain links, are preferably variable in respect of the orientation of their individual polarity only when the magnetic bar has been dismounted from the insertable carriage (5) or basket for items to be washed.

9. Program-controlled equipment washing machine, more especially an automatic washing machine for the laboratory and hospital sector, according to one or more of claims 1 to 8, characterised in that the permanent magnets (8) are disposed in the magnetic bar as separate chain links, which are preferably non-detachable.

10. Program-controlled equipment washing machine, more especially an automatic washing machine for the laboratory and hospital sector, according to one or more of claims 1 to 9, characterised in that each permanent magnet (8) of a magnetic key (6) is disposed in a non-magnetic insert with coupling means (12) provided on both sides, and in that the coupling means (12) engage in one another so as to be rotatable through 180°.

11. Program-controlled equipment washing machine, more especially an automatic washing machine for the laboratory and hospital sector, according to one or more of claims 1 to 10, characterised in that the inserts are interlinked via locking connections (13).

12. Program-controlled equipment washing machine, more especially an automatic washing machine for the laboratory and hospital sector, according to one or more of claims 1 to 11, characterised in that the permanent magnets (8) have a disc-like configuration, and the disposition of the magnetic bar in the basket or insertable carriage (5) is so selected that the orientation of the magnetic field of the magnetic poles for the actuatable magnetic sensors (10) extends transversely relative to the direction of displacement of the insertable carriage (5) or basket for items to be washed.

13. Program-controlled equipment washing machine, more especially an automatic washing machine for the laboratory and hospital sector, according to one or more of claims 1 to 12, characterised in that the magnetic sensors (10) are preferably positioned in a hollow bar (15) beneath the washing chamber base (16) or internally of the appliance door (2) between the internal door panel and the external door panel.

14. Program-controlled equipment washing machine, more especially an automatic washing machine for the laboratory and hospital sector, according to one or more of claims 1 to 13, characterised in that Hall sensors, actuated by the magnetic keys (6), are used as magnetic sensors (10).

15. Program-controlled equipment washing machine, more especially an automatic washing machine for the laboratory and hospital sector, according to one or more of claims 1 to 13, characterised in that microswitches are used as magnetic sensors (10), the switching levers of which carry individual magnets, or the switching levers of which can be influenced directly by magnetic forces.

16. Program-controlled equipment washing machine, more especially an automatic washing machine for the laboratory and hospital sector, according to one or more of claims 1 to 15, characterised in that focussing plates (17) are associated with the magnetic sensors (10) for increasing the magnetic field in the sensor region.

17. Program-controlled equipment washing machine, more especially an automatic washing machine for the laboratory and hospital sector, according to one or more of claims 1 to 16, characterised in that magnetic keys (6), having the same number of permanent magnets (8) as the detectable code patterns, each produce only codes having an identical number of log "1" or log "0", and in that a deviation from this specification is considered to be an indication of an error.

18. Program-controlled equipment washing machine, more especially an automatic washing machine for the laboratory and hospital sector, according to one or more of claims 1 to 17, characterised in that the detectable code patterns are supplemented by a parity bit to form log "1" or to form log "0" in even or uneven parity.

## Revendications

1. Lave-vaisselle programmable, avec un ou plusieurs chariots ou paniers de lavage, en particulier appareil de rinçage automatique pour le secteur des laboratoires et des hôpitaux, pour le nettoyage, la désinfection et le séchage programmés d'articles à laver, tels que des tuyaux respiratoires, des cathéters, des instruments de chirurgie, des pipettes ou analogues, qui doivent être regroupés dans des chariots spécifiques ou dans des paniers de lavage séparés en vue de leur traitement dans l'espace de lavage fermé par une porte de l'appareil automatique, dans lequel chaque chariot ou panier est pourvu d'une clé magnétique, dont la magnétisation détermine le programme de lavage pour les articles à laver chargés dans le panier, caractérisé en ce que la clé magnétique (6) est constituée sur le chariot ou le panier de lavage (5) sous la forme d'une baguette magnétique constituée de plusieurs aimants permanents (8) avec une orientation variable de la polarité individuelle (Nord-Sud/Sud-Nord).

2. Lave-vaisselle programmable, en particulier appareil de rinçage automatique pour le secteur des laboratoires et des hôpitaux, suivant la revendication 1, caractérisé en ce que la clé magnétique (6) est fixée au châssis (7) du chariot ou du panier du côté de la porte de l'appareil et peut être retirée du chariot (5) ou du panier de lavage pour modifier le programme de lavage, et en ce qu'il est prévu, dans la porte (2) de l'appareil ou en dessous de l'ouverture de l'espace de lavage dans la région de la porte (2) de l'appareil, des capteurs magnétiques (10) pouvant être activés par les clés magnétiques (6) et reconnaissant la polarité individuelle respective des aimants permanents (8) d'une clé.

3. Lave-vaisselle programmable, en particulier appareil de rinçage automatique pour le secteur des laboratoires et des hôpitaux, suivant la revendication 1 et 2, caractérisé en ce que les capteurs magnétiques (10) génèrent des modèles de codes à interroger pour la sélection du programme de lavage, qui correspondent aux polarités magnétiques des aimants permanents (8) de la clé magnétique (6).

4. Lave-vaisselle programmable, en particulier appareil de rinçage automatique pour le secteur des laboratoires et des hôpitaux, suivant une ou plusieurs des revendications 1 à 3, caractérisé en ce que des capteurs magnétiques (10) sont présents en nombre égal au nombre des aimants permanents (8) d'une clé magnétique (6).

5. Lave-vaisselle programmable, en particulier appareil de rinçage automatique pour le secteur des laboratoires et des hôpitaux, suivant une ou plusieurs des revendications 1 à 4, caractérisé en ce qu'il est prévu, pour chaque chariot ou panier, un nombre égal d'aimants permanents (8), qui sont cependant polarisés différemment, dans l'orientation de leur polarité individuelle, selon le programme de lavage respectif à sélectionner.

6. Lave-vaisselle programmable, en particulier appareil de rinçage automatique pour le secteur des laboratoires et des hôpitaux, suivant une ou plusieurs des revendications 1 à 5, caractérisé en ce que chaque clé magnétique (6) est fixée de façon amovible au chariot (5) ou au panier de lavage.

7. Lave-vaisselle programmable, en particulier appareil de rinçage automatique pour le secteur des laboratoires et des hôpitaux, suivant une ou plusieurs des revendications 1 à 6, caractérisé en ce que les aimants permanents (8) d'une piste magnétique sont installés en série à la façon d'une chaîne à l'intérieur de la clé magnétique (6) et sont disposés de façon à pouvoir tourner sur eux-mêmes à l'intérieur de l'assemblage en chaîne pour modifier l'orientation de la polarité.

8. Lave-vaisselle programmable, en particulier appareil de rinçage automatique pour le secteur des laboratoires et des hôpitaux, suivant une ou plusieurs des revendications 1 à 7, caractérisé en ce que les aimants permanents (8), en tant qu'éléments de chaîne séparés, ne peuvent de préférence avoir l'orientation de leur polarité individuelle modifiée que si la baguette magnétique est démontée du chariot (5) ou du panier de lavage.

9. Lave-vaisselle programmable, en particulier appareil de rinçage automatique pour le secteur des laboratoires et des hôpitaux, suivant une ou plusieurs des revendications 1 à 8, caractérisé en ce que les aimants permanents (8) sont disposés comme des éléments de chaîne séparés, de préférence indémontables, dans la baguette magnétique.

10. Lave-vaisselle programmable, en particulier appareil de rinçage automatique pour le secteur des laboratoires et des hôpitaux, suivant une ou plusieurs des revendications 1 à 9, caractérisé en ce que chaque aimant permanent (8) d'une clé magnétique (6) est disposé dans un support non magnétique avec des éléments de couplage (12) ménagés de part et d'autre, et en ce que les éléments de couplage (12) s'engagent l'un dans l'autre en pouvant tourner de 180°.

11. Lave-vaisselle programmable, en particulier appareil de rinçage automatique pour le secteur des laboratoires et des hôpitaux, suivant une ou plusieurs des revendications 1 à 10, caractérisé en ce que les supports sont enchaînés l'un à l'autre par des assemblages à encoches (13).

12. Lave-vaisselle programmable, en particulier appareil de rinçage automatique pour le secteur des laboratoires et des hôpitaux, suivant une ou plusieurs des revendications 1 à 11, caractérisé en ce que les aimants permanents (8) sont réalisés en forme de disque et la disposition de la baguette magnétique dans le panier ou le chariot (5) est choisie telle que l'orientation du champ magnétique des pôles magnétiques pour les capteurs magnétiques (10) à activer soit dirigée transversalement à la direction de déplacement du chariot (5) ou du panier de lavage.

13. Lave-vaisselle programmable, en particulier appareil de rinçage automatique pour le secteur des laboratoires et des hôpitaux, suivant une ou plusieurs des revendications 1 à 12, caractérisé en ce que les capteurs magnétiques (10) sont positionnés de préférence dans une baguette creuse (15) en dessous du fond de l'espace de lavage (16) ou à l'intérieur de la porte (2) de l'appareil entre la tôle intérieure de la porte et la tôle extérieure de la porte.

14. Lave-vaisselle programmable, en particulier appareil de rinçage automatique pour le secteur des laboratoires et des hôpitaux, suivant une ou plusieurs des revendications 1 à 13, caractérisé en ce que l'on utilise comme capteurs magnétiques (10) des cellules de Hall activées par les clés magnétiques (6).

15. Lave-vaisselle programmable, en particulier appareil de rinçage automatique pour le secteur des laboratoires et des hôpitaux, suivant une ou plusieurs des revendications 1 à 13, caractérisé en ce que l'on utilise comme capteurs magnétiques (10) des microinterrupteurs, dont les bascules de commutation portent des aimants individuels ou dont les bascules de commutation peuvent être influencées directement par des forces magnétiques.

16. Lave-vaisselle programmable, en particulier appareil de rinçage automatique pour le secteur des laboratoires et des hôpitaux, suivant une ou plusieurs des revendications 1 à 15, caractérisé en ce que des tôles de focalisation (17) sont associées aux capteurs magnétiques (10) pour accroître le champ magnétique dans la région des capteurs.

17. Lave-vaisselle programmable, en particulier appareil de rinçage automatique pour le secteur des laboratoires et des hôpitaux, suivant une ou plusieurs des revendications 1 à 16, caractérisé en ce que des clés magnétiques (6) avec un nombre égal d'aimants permanents (8) génèrent, comme modèles de codes à interroger, uniquement des codes avec un nombre égal de log "1" ou log "0", et en ce que tout écart par rapport à cette prescription est interprété comme un message d'erreur.

18. Lave-vaisselle programmable, en particulier appareil de rinçage automatique pour le secteur des laboratoires et des hôpitaux, suivant une ou plusieurs des revendications 1 à 17, caractérisé en ce que les modèles de codes à interroger sont complétés par un caractère de parité à log "1" ou à log "0" en parité paire ou impaire.
